# EUROPEAN PATENT APPLICATION

(11) **EP 3 903 851 A1**
(43) Date of publication of application: **03.11.2021**
(21) Application number: 20172159.4
(22) Date of filing: 29.04.2020
(51) Int. Cl.: A61M 1/28, A61M 1/16

(54) **PERITONEAL DIALYSIS FLUID FROM CONCENTRATE CONTAINERS**

(71) Applicant: Bellco S.r.l. con Unico Socio, 41037 Modena (IT)
(72) Inventor: Veratti, Andrea, 41122 Modena (IT); Ricci, Mariachiara, 41037 Modena (IT); Petrucci, Alberto, 41037 Modena (IT); Orlandini, Salvatore, 41037 Modena (IT)
(74) Representative: Maschio, Antonio

(57) **Abstract**

The disclosure relates to systems and methods for generating specific peritoneal dialysates from concentrate containers. The systems and methods control an order of adding concentrates from one or more concentrate containers or pouches to generate a peritoneal dialysis fluid. The systems and methods can minimize or avoid formation of a precipitate during the formation of the peritoneal dialysis fluid.

## Description

### FIELD

The disclosure relates to systems and methods for generating peritoneal dialysis fluid from concentrate containers. The systems and methods can control an order of addition of concentrates from separate concentrate containers to accurately generate the peritoneal dialysis fluid to minimize or avoid formation of a precipitate.

### BACKGROUND

In peritoneal dialysis known systems and methods provide pre-packaged bags of peritoneal dialysis fluid. Storage of sufficiently pre-mixed peritoneal dialysis fluid bags requires excess space that may not be available or convenient for some patients. Certain systems and methods generate peritoneal dialysis fluid from liquid concentrates and powder. However, known systems and methods are not capable of generating peritoneal dialysis fluid with solutes in a range of concentrations that can be varied independent of any other solutes. Further, when using separate concentrates for each component of peritoneal dialysis fluid, precipitation can occur during mixing, reducing the accuracy of the final concentrations of components in the peritoneal dialysis fluid. As such, there is a need for systems and methods that can generate peritoneal dialysis fluid with each solute having a specified concentration across a wide range of concentrations from separate concentrate pouches. There is further a need for systems and methods that can accurately generate peritoneal dialysis fluid without the formation of precipitates or the minimization of precipitates.

### SUMMARY OF THE INVENTION

The first aspect of the invention relates to a system. In any embodiment, the system can include a peritoneal dialysis fluid generation flow path, the peritoneal dialysis fluid generation flow path fluidly connectable to a water source and one or more concentrate containers fluidly connectable to the peritoneal dialysis fluid generation flow path, the one or more concentrate containers comprising: at least a concentrate container containing at least one of calcium or magnesium, at least a concentrate container containing at least dextrose, and at least a concentrate container containing at least sodium bicarbonate wherein sodium chloride is present in at least one concentrate container; and a control system programmed to pump water from the water source into each of the one or more concentrate containers to generate one or more concentrates, the control system also being programmed to subsequently pump the one or more generated concentrates into the mixing container, wherein the control system is programmed to sequentially pump a dextrose concentrate from the concentrate container containing dextrose into the mixing container in between: pumping an ion concentrate from the concentrate container containing at least calcium or magnesium into the mixing container and pumping a sodium bicarbonate concentrate from the concentrate container containing sodium bicarbonate into the mixing container, or in between: pumping a sodium bicarbonate concentrate from the concentrate container containing sodium bicarbonate into the mixing container and pumping an ion concentrate from the concentrate container containing at least calcium or magnesium into the mixing container.

In any embodiment, one or more filters can be in the peritoneal dialysis fluid generation flow path.

In any embodiment, the concentrate container containing sodium bicarbonate can also have sodium lactate.

In any embodiment, the concentrate container containing sodium bicarbonate can also have sodium chloride.

In any embodiment, at least one of the concentrate containers can contain a solid substance.

In any embodiment, at least one of the concentrate containers can have a liquid or aqueous solution.

In any embodiment, the system can have one or more valves directing fluid between the one or more concentrate containers and the mixing container.

In any embodiment, wherein the water pumped into the one or more concentrate containers can be purified water.

In any embodiment, the system can have a water purification module downstream of the water source and in fluid communication with the water source.

In any embodiment, wherein the water pumped into the one or more concentrate containers can be sterile water.

In any embodiment, the system can also have a water sterilization module downstream of the water source and in fluid communication with the water source.

In any embodiment, the system can have one or more filters in the peritoneal dialysis fluid generation flow path.

In any embodiment, the system can have one or more filters positioned upstream of the mixing container.

In any embodiment, the system can have a peritoneal dialysis cycler in fluid connection with the mixing container.

In any embodiment, the one or more concentrate containers can be a pouch.

The features disclosed as being part of the first aspect of the invention can be in the first aspect of the invention, either alone or in combination, or follow any arrangement or permutation of any one or more of the described elements. Similarly, any features disclosed as being part of the first aspect of the invention can be in a second aspect of the invention described below, either alone or in combination, or follow any arrangement or permutation of any one or more of the described elements.

The second aspect of the invention is drawn to a method. In any embodiment, the method can include the steps of pumping water from a water source into one or more concentrate containers to generate a concentrate; the one or more concentrate containers having at least a concentrate container containing at least one of calcium or magnesium, at least a concentrate container containing at least dextrose, and at least a concentrate container having at least sodium bicarbonate; wherein sodium chloride can be present in at least one concentrate container; and pumping a dextrose concentrate from the concentrate container containing dextrose into the mixing container in between pumping an ion concentrate from the concentrate container calcium or magnesium into the mixing container and pumping a sodium bicarbonate concentrate from the concentrate container containing sodium bicarbonate into the mixing container, or pumping the dextrose concentrate from the concentrate container containing dextrose in between pumping a sodium bicarbonate concentrate from the concentrate container containing sodium bicarbonate into the mixing container and pumping an ion concentrate from the concentrate container containing calcium or magnesium into the mixing container.

In any embodiment, the method can also have the step of pumping water into the mixing container.

In any embodiment, the concentrate container containing sodium bicarbonate can also have sodium lactate.

In any embodiment, the water pumped into the one or more concentrate containers can be purified water.

In any embodiment, wherein the water pumped into the one or more concentrate containers can be sterile water.

In any embodiment, the method can also have the step of pumping water into the mixing container after the step of pumping one or more concentrates from the one or more concentrate containers.

In any embodiment, the method can also have the step of filtering fluid prior to adding the concentrates to the mixing container.

In any embodiment, the method can also have the step of pumping fluid inside the mixing container to a peritoneal dialysis cycler.

In any embodiment, the one or more concentrate containers can be a pouch.

The features disclosed as being part of the second aspect of the invention can be in any other part of the second aspect of the invention, either alone or in combination, or follow any arrangement or permutation of any one or more of the described elements. Similarly, any features disclosed as being part of the second aspect of the invention can be in the first aspect of the invention, either alone or in combination, or follow any arrangement or permutation of any one or more of the described elements.

### BRIEF DESCRIPTION OF THE DRAWINGS

FIG. 1 is a detailed view of a peritoneal dialysis system.
FIG. 2 is an experimental setup to test a peritoneal dialysis generation system.
FIG.'s 3A-B illustrate a concentrate pouch. FIG. 3A is a perspective view of a cutaway showing one side of the concentrate pouch. FIG. 3B is an exterior side view of the concentrate pouch.

### DETAILED DESCRIPTION

Unless defined otherwise, all technical and scientific terms used have the same meaning as commonly understood by one of ordinary skill in the art.

The articles "a" and "an" are used to refer to one to over one (i.e., to at least one) of the grammatical object of the article. For example, "an element" means one element or over one element.

The terms "aqueous," "aqueous substance," or an "aqueous solution" refer to a solution of a solute in water.

The term "at least" refers to a possibility of one or more elements may be present. For example, a container containing at least magnesium provides for the possibility of containing one or more other components such as calcium.

The term "between" when referring to the performance of certain events, refers to one or more sequence, step, or period after a first event, but before a second event. That is, the one or more events that occur after the first event but before the second event can be considered to be "between" the first and second events. The first and second events can occur in any order, with the term "between" merely referring to the sequence or time after one event and before the other event.

The term "calcium," as used herein, can refer to any source of calcium, such as calcium chloride.

The term "completely dissolve" refers to the process of generating a solution from a solute in which essentially all or no solute remains undissolved.

A "component" of peritoneal dialysis fluid refers to any solute dissolved in water for use in peritoneal dialysis.

The term "comprising" includes, but is not limited to, whatever follows the word "comprising." Use of the term indicates the listed elements are required or mandatory but that other elements are optional and may be present.

A "concentrate" refers to a solution of one or more solutes in water. The concentrate can have a solute concentration greater than that to be used in dialysis of any type including peritoneal dialysis and hemodialysis.

The term "concentrate pouch" refers to a container that initially contains a concentrate fluid to be formed or contained therein. The pouch can be made of any suitable material and can contain dry, wet, or a combination of dry and wet materials.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

The terms "contain" or "containing" refer to a material held within a component or container. The term contain is open ended and does not prevent the inclusion of other components being included within the same component.

The terms "control," "controlling," or "controls" refer to the ability of one component to direct the actions of one or more second or other components.

A "control system" can be a device that monitors and affects the operational conditions of a given system. The operational conditions are typically referred to as output variables of the system wherein the output variables can be affected by adjusting certain input variables. The control system can be any number or combination of processors, controllers, software, and computers.

The term "dextrose" refers to a simple sugar compound with a chemical formula C₆H₁₂O₆. Dextrose is the dextrorotatory form of glucose, and in certain embodiments, the terms dextrose and glucose can be used interchangeably.

The term "dextrose concentrate" refers to a solution containing at least dextrose which has been dissolved from solid dextrose or a dextrose solution formed from a concentrated dextrose solution. The concentrated dextrose solution can also be referred to as a "generated dextrose concentrate."

The term "dissolve" refers to the process of dissolution or more broadly, the generation of a solution from a solvent and solute.

The term "downstream" refers to a position of a first component in a flow path relative to a second component wherein fluid will pass by the second component prior to the first component during normal operation. The first component can be said to be "downstream" of the second component, while the second component is "upstream" of the first component.

The term "filter," as used herein, refers to a device inhibiting passage of microbes or fragments of microbes such as endotoxins in a fluid or solution while allowing the passage of the fluid or solution.

The term "filtering" refers to a process of separating certain solid components such as, but not limited to biological or chemical contaminants from liquids or gases using a filter medium that allows the fluid to pass through, but not the biological or chemical contaminants.

The terms "first," "second," and "third," and the like, refer to separate and distinct features. For example, one or more pouches can be identified as a 'first concentrate pouch," "second concentrate pouch," and "third concentrate pouch."

A "fluid" is a liquid substance optionally having a combination of gas and liquid phases in the fluid. Notably, a liquid can have a mixture of gas and liquid phases of matter.

A "fluid path" or "fluid line" can refer to a tubing or conduit through which a fluid, gas, or a combination thereof can pass. The fluid path or line can also contain air during different modes of operation such as cleaning or purging of a path or line.

The term "fluidly connectable" refers to the ability to provide passage of fluid, gas, or combinations thereof, from one point to another point. The ability to provide such passage can be any mechanical connection, fastening, or forming between two points to permit the flow of fluid, gas, or combinations thereof. The two points can be within or between any one or more of compartments, modules, systems, components, and rechargers, all of any type. Notably, the components that are fluidly connectable, need not be a part of a structure. For example, an outlet "fluidly connectable" to a pump does not require the pump, but merely that the outlet has the features necessary for fluid connection to the pump.

The term "fluidly connected" refers to a particular state or configuration of one or more components such that fluid, gas, or combination thereof, can flow from one point to another point. The connection state can also include an optional unconnected state or configuration, such that the two points are disconnected from each other to discontinue flow. It will be further understood that the two "fluidly connectable" points, as defined above, can form a "fluidly connected" state. The two points can be within or between any one or more of compartments, modules, systems, components, all of any type.

An "fluid inlet" is a portion of a component through which gas, fluid, and combinations thereof can enter or exit the component. Although the term inlet generally refers to an opening for entry of gas, fluid, and combinations thereof, the inlet can sometimes provide a means for exiting or exhausting the gas, fluid, and combinations thereof. For example, during a priming, cleaning, or disinfection, the inlet can be used to remove gas, fluid, and combinations thereof through the inlet. Also, during operation, the inlet can remove gas, fluid, and combinations thereof.

The phrases "generate a concentrate" or "generating a concentrate" refer to a process or steps for dissolving a solid or liquid material, or diluting a solution, to form a concentrated solution.

The terms "initial" or "initially" in reference to feature or object refers to a beginning or starting condition or state of the feature. For example, a "concentrate container initially containing a solid substance" refers to the container starting with a solid substance wherein a "subsequent" state or condition of the concentrate container can change, such as where a solid material is reconstituted by the addition of water. Similarly, the term "subsequent" refers to a following state or condition of the feature or object.

The term "ion concentrate" refers to a solution containing one or more ions being dissolved from a solid ion or being formed from a concentrated ionic solution. The concentrated ion solution can also be referred to as a "generated ion concentrate."

The term "liquid," "liquid substance," or a "liquid material" refer to a material in the liquid phase of matter.

The term "magnesium," as used herein, can refer to any source of magnesium, such as magnesium chloride.

A "mixing container" is a container into which one or more fluid streams can be flowed to generate a mixture. The mixing can occur by passive turbulence or active means such as an agitator.

"Peritoneal dialysis fluid" is a dialysis solution to be used in peritoneal dialysis having specified parameters for purity and sterility. Peritoneal dialysis fluid is not the same as dialysate fluid of the type used in hemodialysis.

"Peritoneal dialysis" is a therapy wherein a peritoneal dialysis fluid is infused into the peritoneal cavity, which serves as a natural dialyzer. In general, waste components diffuse from a patient's bloodstream across a peritoneal membrane into the dialysis solution via a concentration gradient. In general, excess fluid in the form of plasma water flows from a patient's bloodstream across a peritoneal membrane into the dialysis solution via an osmotic gradient. Once the infused peritoneal dialysis solution has captured sufficient amounts of the waste components the fluid is removed. This cycle can be repeated for several cycles each day or as needed.

A "peritoneal dialysis cycler" is a component for movement of fluid into and out of the peritoneal cavity of a patient.

A "peritoneal dialysis fluid generation flow path" is a fluid flow path that can receive fluids, concentrates, and/or water for the generation of a peritoneal dialysis fluid.

The term "positioned" refers to a component, feature, or process step connected to or in relation to the feature being referred to, or the relative location with respect thereto. The contact can be physical, fluid, temporal, or electrical and is intended to be used in the broadest reasonable sense.

The term "pouch" can refer to a flexible bag of any size and shape fabricated from a suitable material to hold any one of the concentrates of the invention.

The term "prior" or "before" with respect to steps in an algorithm or control process refers to one or more step or sequence of steps preceding the referred step.

The term "processor" or "microprocessor" as used are broad terms and are to be given the ordinary and customary meaning for a person of ordinary skill in the art. The terms refer without limitation to any computer system, state machine, processor, or the like designed to perform arithmetic or logic operations using logic circuitry that responds to and processes the basic instructions that drive a computer. The terms can include ROM ("read-only memory") and/or RAM ("random-access memory") associated therewith.

The term "programmed" is to be interpreted in the broadest sense and can mean any series of instructions that cause a processor or a control system of any type including software to perform certain steps or to execute an algorithm of any type. For example, a processor can be programmed to execute an algorithm. Alternatively, a computer can be programmed to execute a series of functions of steps.

The terms "pumping," "pumped," or to "pump" refer to moving or flowing a fluid using a pump of any type known to those of ordinary skill in the art.

The terms "pumping fluid" or to "pump fluid" refer to moving a fluid, gas, or a combination thereof through a flow path of any type with a pressure. The pressure can be generated by a pump of any type.

The term "purified water" refers to any water treated to remove at least some biological or chemical contaminants. In one non-limiting embodiment, the term "purified water" can refer to the definition of purified water in United States Pharmacopeia, 23d Revision, January 1, 1995.

The terms "sequential" or "sequentially" refer to forming or following a specified ordered or arranged series of steps or functions of any type.

"Sodium bicarbonate" refers to NaHCO₃, either in solution or solid form.

"Sodium chloride" refers to NaCl, either in solution or solid form.

"Sodium lactate" refers to C₃H₅NaO₃, either in solution or solid form.

The term "sodium lactate concentrate" refers to a solution containing at least sodium lactate which has been dissolved from solid sodium lactate or a sodium lactate solution formed from a concentrated sodium lactate solution. The concentrated sodium lactate solution can also be referred to as a "generated sodium lactate concentrate."

The terms "solid," "solid substance," or a "solid material" refer to a material in the solid phase of matter, and can include crystalline, powdered, or any other form of solid material.

The terms "subsequently," "subsequent", and the like, refer to a step, point, or function later in time, place, or position.

The term "sterile water" refers to water that is generally free of all micro-organisms or water in which no viable bacteria, viruses, or other type of viable microorganisms are present.

The term "upstream" refers to a position of a first component in a flow path relative to a second component wherein fluid will pass by the first component before the second component during normal operation. The first component can be said to be "upstream" of the second component, while the second component is "downstream" of the first component.

The term "valve" refers to any mechanism or structure that can open and close to control the flow of any number of fluids. For example, the valve can be a 2-way, 3-way, or any combination of open and closed states to control flow.

The term "water purification module" refers to a component or components capable of removing biological and/or chemical contaminants from water.

The term "water source" refers to a source from which any one of potable, purified, or sterile water can be obtained.

The term "water sterilization module" can be a component that sterilizes water by any process including filtration, irradiation, or any other known methods known to those of skill in the art.

### Peritoneal Dialysis Generation System

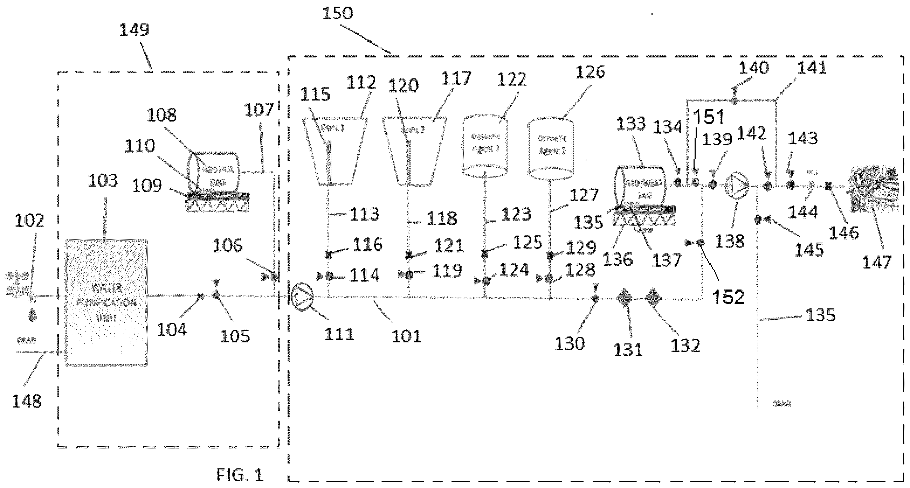

FIG. 1 illustrates a non-limiting example of a peritoneal dialysis system having a control system. The control system can be programmed to pump water from a water source **102** or a purified water reservoir **108** into each of one or more concentrate containers to generate a concentrate solution or fluid. The concentrate containers can include but are not limited to a first concentrate container **112,** a second concentrate container **117,** a first osmotic agent container **122,** and a second osmotic agent container **126.** Each of the concentrate containers can generate one or more concentrate solutions. The control system can be programmed to subsequently pump the one or more generated concentrates from the one or more concentrate containers into a mixing container **113** to avoid precipitation. The control system can be programmed to sequentially pump a dextrose concentrate from either the first osmotic agent container **122** or the second osmotic agent container **126** into the mixing container **113** in between the step of pumping an ion concentrate from a concentrate container containing at least calcium or magnesium into the mixing container **113** and the step of pumping a sodium bicarbonate concentrate from a concentrate container containing sodium bicarbonate into the mixing container **113.** The control system can also be programmed to sequentially pump a dextrose concentrate from either the first osmotic agent container **122** or the second osmotic agent container **126** into the mixing container **113** in between the step of pumping a sodium bicarbonate concentrate from the concentrate container containing sodium bicarbonate into the mixing container **113** and the step of pumping an ion concentrate from a concentrate container into the mixing container **113.** Notably, the algorithm avoids precipitation by pumping dextrose concentrate into the mixing container **113** in between the steps of pumping in the ion concentrate solution and the sodium bicarbonate solution, regardless of whether the ion concentrate solution is pumped first, or the sodium bicarbonate solution is pumped first.

Water from the water source **102** can be purified by a water purification unit **103.** The water purification unit **103** can remove chemical and biological contaminants from the source water **102.** Tap water or other similar water sources can be the water source **102.** The water source **102** can be used to generate a peritoneal dialysis fluid if suitable sterilization mechanisms know to those of ordinary skill are positioned in the system. Preferably, the suitable sterilization mechanisms would be positioned prior to the final delivery of the peritoneal dialysis fluid to a patient **147.** The water purification unit **103** can be any component capable of removing one or more contaminants from water obtained from the water source **102.** The water purification unit **103** can be any suitable device, mechanism or procedure known to those of skill in the art including, but not limited to any one or more of a sorbent cartridge, reverse osmosis module, nanofilter, combination of ion and anion exchange materials, activated carbon, silica, or silica based columns. Optionally, a water sterilization module (not shown) can be provided in addition to the water purification unit **103** to generate sterile water generally free of viable microbiological contaminants. The peritoneal dialysis system can alternatively have a direct connection to a purified or sterile water source (not shown) such as pre-packaged purified or sterile water, in which case the water purification unit **103** or water sterilization module (not shown) can be omitted or bypassed. Drain line **148** can drain any waste water from water purification unit **103.** Alternatively, a purified water source can be used without the water purification unit **103.**

The purified water can be pumped into a fluid line **101.** Valve **105** directs the movement of fluid from the water source **102** and water purification unit **103** into the peritoneal dialysis fluid generation portion of the system. In certain embodiments, purified water can be directed to purified water reservoir **108.** In other embodiments, sterile water can be used where the concentrates are sterile. Valve **106** can be opened to allow purified water through fluid line **107** into water reservoir **108.** A heater **109** can heat the purified water, to speed dissolution of dry powders used to generate peritoneal dialysis fluid. A temperature sensor **110** can be included to determine the temperature of the purified water in water reservoir **108.** Alternatively, the heater **109** can act as an inline temperature sensor. One of skill in the art will understand that the sensor locations illustrated in FIG. 1 are for illustrative purposes only because other suitable locations for the sensors can be envisioned by those of ordinary skill. A control system (not shown) can be in communication with the temperature sensor **110** and can control the heater **109** to heat the water to a desired temperature. To begin generating the peritoneal dialysis fluid, water from water reservoir **108** can be added to the fluid line **101** of the peritoneal dialysis fluid generation system through valve **106** and fluid line **107.** Pump **111** can provide the driving force necessary to move fluid throughout the system.

In particular, the first concentrate container **112,** the second concentrate container **117,** the first osmotic agent container **122,** and the second osmotic agent container **126** are fluidly connected or fluidly connectable to a fluid line **101.** Although shown as having four concentrate described as the first concentrate container **112,** the second concentrate container **117,** the first osmotic agent container **122,** and the second osmotic agent container **126,** the peritoneal dialysis system of the invention can use any combination of two or more concentrate containers where permutations of concentrates and osmotic agents can be distributed between the various containers as described herein. In certain embodiments, the first concentrate container **112** can contain at least one of calcium or magnesium. One or more other excipients except for sodium bicarbonate can be contained inside the first concentrate container **112.** The first osmotic agent container **122** can contain at least dextrose. One or more additional osmotic agents, such as icodextrin or other osmotic agents, can be contained inside container **122,** or optionally in second osmotic agent container **126.** The second concentrate container **117** can contain at least sodium bicarbonate, and optionally sodium lactate and sodium chloride. Alternatively, the sodium chloride and sodium lactate can be contained in any of the other concentrate containers. Although FIG. 1 illustrates the first concentrate container **112** and second concentrate container **117** as concentrate pouches with first osmotic agent container **122** and a second osmotic agent container **126** as containers for containing a liquid substance, any of the concentrate containers can be of any shape or type and can initially contain solid, powder, or liquid substances or an aqueous solution.

Depending on the particular embodiment, all of the concentrate containers can contain a solid substance; all of the concentrate containers can contain a powder; and, all of the concentrate containers can contain a liquid or aqueous solution. Alternatively, the concentrate containers can contain any combination of solid, powder, or liquid substances or aqueous solution. In particular, at least one of the concentrate containers can initially contain a solid substance such as a powder. In another embodiment, at least one of the concentrate containers initially contains a liquid or aqueous solution. The physical arrangement of the various containers can vary, and be positioned in any order, so long as a control algorithm controls the flow and order of fluid flow using any number of valves and fluid lines, as provided herein. Hence, one of ordinary skill will understand that the designation of a first, second, third, fourth container, and the like are to be interpreted in the broadest sense and is not intended to limit the physical placement or location of the containers, but rather to only identify a particular container.

A first connector **116,** a second connector **121,** a third connector **125,** and a fourth connector **129** allow for the first concentrate container **112,** the second concentrate container **117,** the first osmotic agent container **122,** and the second osmotic agent container **126** to be reversibly attached and detached from the fluid line **101.** Any combination of connectors and sets of connectors to the respective pouches can be configured to provide for modular attachment and detachment of the containers to the fluid line **101.**

Upon attachment to fluid line **101,** a valve **114** can be opened, and a valve **119,** a valve **124,** a valve **128,** and a valve **130** can be closed to allow water through fluid line **113** and into the first concentrate container **112** through fluid inlet tube **115.** The water can dissolve a solid or liquid material inside the first concentrate container **112** or dilute a concentrated aqueous solution initially present in the first concentrate container **112.** Valve **114** can be closed, and valve **119** opened to allow water through fluid line **118** and into the second concentrate container **117** through a fluid inlet tube **120.** The water can dissolve or dilute a solid, liquid, or aqueous material in the second concentrate container **117.** Valve **119** can be closed and valve **124** can be opened to allow water through fluid line **123** and into the first osmotic agent container **122** to dissolve or dilute a solid, liquid, or aqueous material in first osmotic agent container **122.** Valve **124** can be closed and valve **128** opened to allow water through fluid line **127** and into second osmotic agent container **126** to dissolve or dilute a solid, liquid, or aqueous material in second osmotic agent container **126.** As described, the osmotic agent containers **122** and **126** can be similar to concentrate containers **112** and **117,** including inlet tubes not illustrated in FIG. 1.

The system can similarly operate any combination and order of valves to selectively flow water into any of the first concentrate container **112,** the second concentrate container **117,** the first osmotic agent container **122,** and the second osmotic agent container **126.** Alternative concentrate container combinations can be used having any number of concentrate containers, including 1, 2, 3, 4, or more concentrate containers. In certain embodiments, two separate concentrate containers containing different osmotic agents can be used, such as a first concentrate container containing dextrose and a second concentrate container containing an alternative osmotic agent, such as icodextrin. In other embodiments, only a single osmotic agent container can be included. The containers can optionally be a flexible pouch made from any suitable material known to those of skill in the art.

To generate peritoneal dialysis fluid, valve **114,** valve **130,** valve **134,** valve **139,** valve **140,** valve **142,** and valve **152** can be opened with valve **143,** valve **145,** and valve **151** closed to pump a concentrate solution from concentrate container **112** into fluid line **101** and fluid line **141** and then pump the concentrate solution into a mixing container **133** After passing through valve **130,** the concentrate solution can pass through one or more filter **131** and filter **132** to sterilize the fluid before being added to the mixing container **133.** The filters can be hollow fiber filters or flat filters, or any other type of filters known in the art. The filters can filter out contaminants such that the resulting filtered fluid is free of any viable microbiological components or otherwise meets relevant regulatory standards for a peritoneal dialysis fluid. Valve **114** can be closed, and valve **119** opened to pump a concentrate solution from concentrate container **117** into fluid line **101** and then into mixing container **133.** Closing valve **119** and opening valve **124** allows concentrate from concentrate container **122** to be added to mixing container **133.** Alternatively, or additionally, a different osmotic agent can be added to mixing container **133** by opening valve **128.** In certain embodiments, water can be pumped to mixing container **133** from water reservoir **108** while the individual concentrates are being added to mixing container **133** and between additions of the various concentrates. Adding the concentrates from the first concentrate container **112,** second concentrate container **117,** and first osmotic agent container **122,** and second osmotic container **126** in a specified sequential order can avoid precipitation during preparation of the peritoneal dialysis fluid. One of ordinary skill will understand that the physical arrangement of the first concentrate container **112,** second concentrate container **117,** and first osmotic agent container **122,** and second osmotic container **126** can vary, and be positioned in any physical location, so long as a control algorithm controls the flow and order of fluid flow using any number of valves and fluid lines, as provided herein. Hence, one of ordinary skill will understand that the designation of a first, second, third, fourth container, and the like are to be interpreted in the broadest sense and is not intended to limit the physical placement or location of the containers.

In mixing container **133,** the concentrate solutions from each of the first concentrate container **112,** the second concentrate container **117,** the first osmotic agent container **122,** and the second osmotic agent container **126** can be mixed with purified water to generate the peritoneal dialysis fluid. The peritoneal dialysis fluid can be heated with heater **136** as measured by temperature sensor **137.** The control system can control the heater **136** based on data from temperature sensor **137** to heat the peritoneal dialysis fluid to a desired temperature prior to infusion into the patient **147.**

Cycler pump **138** can control the cycling of peritoneal dialysis fluid into and out of the patient **147.** To infuse fluid into the patient **147,** valve **134,** valve **139,** valve **142,** valve **143,** and valve **151** can be opened, valve **130,** valve **145,** and valve **152** closed, and cycler pump **138** operated to infuse the peritoneal dialysis fluid into the patient **147.** Pressure sensor **144** can be included to control the fluid pressure of peritoneal dialysis fluid entering the patient and to control a fill volume. To drain used peritoneal dialysis fluid from the patient **147,** valve **142,** valve **152,** and **134** can be closed and valve **139,** valve **140,** valve **143,** valve **145,** and **151** can be opened. Drain fluid can then pass through line 141 and then pumped by pump 138 to drain line **135.** Line **141** can allow pump **138** to pump fluid in the same direction during filling and draining phases.

Any of the concentrate containers, reservoirs, or mixing bags of the system can be positioned on scales to determine the volume of fluid moved into or out of the containers. Alternatively, any other method for determining the amount of fluid moved into or out of the containers can be used. For example, flow sensors, balancing chambers, and/or volumetric pumps can be included to determine the amount of fluid moved into or out of each of the containers, reservoirs, or concentrate containers.

In certain embodiments, portions of the peritoneal dialysis system can be contained within separate modules. For example, the water purification unit **102** can be separated from the peritoneal dialysis fluid generation portions in water purification module **149,** fluidly connectable to a peritoneal dialysis fluid generation system and cycler module **150** through connector **104.** The peritoneal dialysis fluid generation portions of the system can connect to the patient through connector **146.** Separating the system into a water purification module **149** and a peritoneal dialysis fluid generation system and cycler module **150** can allow the water purification components to be located near a water source, while the peritoneal dialysis generation and treatment components can be placed at a location more convenient for treatment. However, the various portions of the peritoneal dialysis system can be contained in alternative modular arrangements, or as a single integrated system.

The heaters, pumps, and valves of the system can be controlled by a control system programmed to generate peritoneal dialysis fluid. The control system can be one or more devices, processors, logic, circuits, or programmable computers which monitor and affect the operational conditions of the system. The control system can open and close the described valves to direct flow inside and through the fluid lines and into or out of any of the described containers. The control system can direct actions of a functional component, such as the one or more pumps and valves via signals transferred between the control system and the functional component. The control system can communicate with the valves, pumps, and sensors by receiving from and sending signals to the valves, pumps, and sensors. The control system can receive input from outside the system, such as a peritoneal dialysis prescription from a user, electronic health records and information, or any other source.

The control system can include programmed instructions to carry out the steps of generating peritoneal dialysis fluid from concentrate containers. The control system can use instructions embedded or encoded in a computer system-readable medium, including a computer system-readable storage medium, and can cause one or more programmable processors, or other processors, to implement one or more of the techniques described herein. The process steps can include instructions included or encoded in the computer system-readable medium. The process steps can be executed by the one or more processors. Computer system readable storage media may include random access memory (RAM), read only memory (ROM), programmable read only memory (PROM), erasable programmable read only memory (EPROM), electronically erasable programmable read only memory (EEPROM), flash memory, a hard disk, a compact disc ROM (CD-ROM), a floppy disk, a cassette, magnetic media, optical media, or other computer system readable media.

The order of addition of concentrates to the mixing container can be controlled by the control system to avoid precipitation. Certain combinations of addition of concentrates result in precipitate formation, reducing accuracy of the peritoneal dialysis fluid.

### Precipitation Experiments

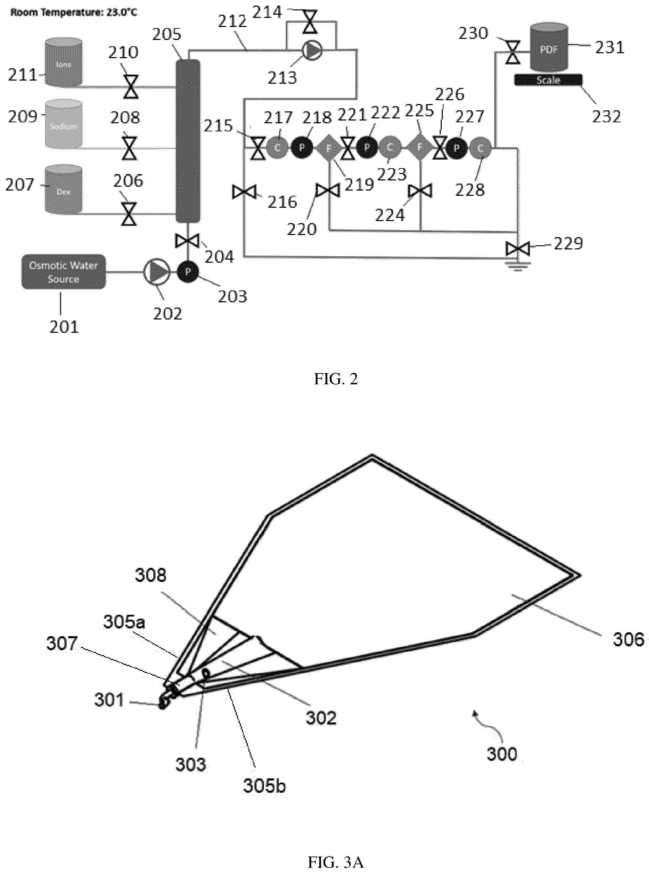

To determine whether a precipitate will form, an experiment was set up as illustrated in FIG. 2, which is a similar system to that illustrated in FIG. 1. A dextrose concentrate was placed in concentrate container **207,** a sodium-based solution/concentrate was placed in concentrate container **209,** and a concentrate containing magnesium chloride and calcium chloride was placed in concentrate container **211.** The system was primed by first opening valve **206** and valve **216** and operating pump **213** at a rate of 50 mL/min. After one minute, valve **206** was closed and valve **208** opened. After an additional minute, valve **208** was closed and valve **210** opened. After one minute, valve **210** was closed and valve **204** and valve **214** opened. Pump **202** was operated at 300 mL/min to pump purified water from osmotic water source **201** while pump **213** was operated at 50 mL/min. All valves were closed. Purified water and the concentrate streams can flow through optional manifold **205** during operation. Fluid line **212** can connect the optional manifold **205** to valve **214** and/or pump **213.** Alternatively, manifold **205** can be replaced with fluid lines and T-connectors.

Next, to prime microbial filter **219** and microbial filter **225,** a valve **204,** a valve **229,** a valve **215,** and a valve **220** were opened. Purified water from osmotic water source **201** was pumped through filter **219** at 300 mL/min for 50 seconds. Valve **220** was closed and valve **221** and valve **224** were opened. Purified water was pumped through both microbial filter **219** and microbial filter **225** at 300 mL/min for 50 seconds. Valve **224** was closed, and valve **226** opened and purified water was pumped through microbial filter **219** and microbial filter **225** at 300 mL/min for 50 seconds.

To fill the peritoneal dialysis fluid bag **231,** all valves were initially closed. Valve **206,** valve **215,** valve **221,** valve **226,** and valve **230** were opened and pump **213** operated to pump a predetermined amount of dextrose concentrate from concentrate container **207** to peritoneal dialysis fluid bag **231.** A scale **232** was used to determine the amount of the concentrate added to peritoneal dialysis fluid bag **231.** Valve **206** was closed and valve **210** opened to pump a predetermined amount of magnesium chloride and calcium chloride concentrate to peritoneal dialysis fluid bag **231.** Next, valve **210** was closed and valve **208** opened to pump a predetermined amount of sodium-based solution concentrate to peritoneal dialysis fluid bag **231.** Valve **208** was closed and valve **204** opened to pump purified water into peritoneal dialysis fluid bag **231.** Samples were then taken from peritoneal dialysis fluid bag **231** to determine the final concentrations of peritoneal dialysis components. In the case of the experiment described, the measured values for sodium, glucose, chloride, calcium, and magnesium varied from the expected measurements by over 5%. Pressure sensor **203,** pressure sensor **218,** pressure sensor **222,** and pressure sensor **227** can be included in any embodiment of the invention to monitor pressure drops across the microbial filter **219** and microbial filter **225** and throughout the system. Conductivity sensor **217,** conductivity sensor **223,** and conductivity sensor **228** can be included in any embodiment of the invention to measure the conductivity of the concentrates as the fluid moves through the system.

As described, certain orders of addition of concentrates to the peritoneal dialysis fluid bag **231** result in invalid results, or formation of a precipitate. Table 1 provides several possible orders of addition with results from the experiments.

**Table 1**

| Stream 1 | Stream 2 | Stream 3 | Stream 4 | Valid |
|---|---|---|---|---|
| WATER | IONS | SODIUM | DEXTROSE | NO |
| IONS | WATER | SODIUM | DEXTROSE | YES |
| SODIUM | WATER | IONS | DEXTROSE | YES |
| WATER | SODIUM | IONS | DEXTROSE | NO |
| IONS | SODIUM | WATER | DEXTROSE | NO |
| SODIUM | IONS | WATER | DEXTROSE | NO |
| SODIUM | IONS | DEXTROSE | WATER | NO |
| IONS | SODIUM | DEXTROSE | WATER | NO |
| DEXTROSE | SODIUM | IONS | WATER | NO |
| SODIUM | DEXTROSE | IONS | WATER | YES |
| IONS | DEXTROSE | SODIUM | WATER | YES |
| DEXTROSE | IONS | SODIUM | WATER | NO |
| DEXTROSE | WATER | SODIUM | IONS | NO |
| WATER | DEXTROSE | SODIUM | IONS | NO |
| SODIUM | DEXTROSE | WATER | IONS | YES |
| DEXTROSE | SODIUM | WATER | IONS | YES |
| WATER | SODIUM | DEXTROSE | IONS | YES |
| SODIUM | WATER | DEXTROSE | IONS | YES |
| IONS | WATER | DEXTROSE | SODIUM | YES |
| WATER | IONS | DEXTROSE | SODIUM | YES |
| DEXTROSE | IONS | WATER | SODIUM | YES |
| IONS | DEXTROSE | WATER | SODIUM | YES |
| WATER | DEXTROSE | IONS | SODIUM | NO |
| DEXTROSE | WATER | IONS | SODIUM | NO |

The fourth column in Table 1 indicates whether the specified order of addition resulted in a valid peritoneal dialysis fluid having all solutes within a specified range of the expected value. The boxes labeled "ions" refer to a concentrate having magnesium chloride and calcium chloride. The boxes labeled "dextrose" refer to a concentrate solution containing dextrose. The boxes labeled "sodium" refer to a concentrate having sodium bicarbonate, and if present, sodium chloride and/or sodium lactate. The boxes labeled "water" refer to addition of purified water.

As indicated in Table 1, several possible orders of addition can be used. Any of the four components tested, dextrose, sodium, magnesium and calcium, or water can be added to the mixing container first. To avoid precipitation, the system can pump dextrose solution into the peritoneal dialysis fluid generator system in between the steps of pumping ions solution from an ion concentrate container and bicarbonate from the sodium concentrate container. Precipitation can be avoided by not adding the sodium immediately preceding or immediately after the magnesium and calcium concentrates. Either water or dextrose can be added to the mixing container between the sodium concentrate and the magnesium and calcium concentrate. The filters can contain a small amount of fluid passing through. If a sodium bicarbonate concentrate immediately precedes or follows a magnesium and calcium concentrate, precipitation may occur within the filters. By passing a different fluid through the filters between the sodium and ion concentrates, the small amount of fluid remaining in the filters may be washed out. Further, although not shown in Table 1, adding water to the mixing container immediately after adding dextrose can help wash the filters, avoiding pressure buildup.

The control system can be programmed to add the concentrates to a mixing container in a specified order to avoid precipitate formation. In particular, the control system can be programmed to sequentially pump a dextrose concentrate into the mixing bag in between the steps of pumping a concentrate containing magnesium or chloride and pumping a sodium stream concentrate. Alternatively, the control system can pump the dextrose solution in between the steps of pumping the sodium stream concentrate and pumping a concentrate of magnesium or chloride into the mixing bag.

### Peritoneal Dialysis Pouches

In certain embodiments, the system can generate peritoneal dialysis fluid from concentrates contained in concentrate containers wherein the containers can be in the form of a flexible pouch. FIG. 3A is a perspective view depicting a cutaway of an interior side **306** of a peritoneal dialysis concentrate pouch **300.** The concentrate pouch **300** can be made from a flexible material formed from two pieces where the pouch side **306** can be welded to another pouch side (not shown). The pouches and/or containers can also be semi-flexible, semi-rigid, or rigid and be fabricated from any suitable process known to those of ordinary skill. The concentrate containers or concentrate pouches of the invention can be constructed from any suitable material. For example, the pouches of the invention can be constructed from mono or multilayer film material, including polypropylene-polyethylene or polypropylene-polyethylene-polyamide for multilayer polyolefin-based films; a monolayer polyolefin film such as polypropylene or polyethylene, or vinyl-based films such as PVC or EVA. Alternatively, other suitable materials can be used. One of ordinary skill will understand that the pouch can be made from any suitable material depending on any desired manufacturing, usage, and size constraints.

The concentrate pouch **300** can initially contain a solid, liquid, or concentrated material. Upon addition of a fluid such as water, the initial contents can change into a different phase of matter. For example, a solid concentrate inside the concentrate pouch **300** can be reconstituted by adding water. Fluid can be pumped into an interior of the concentrate pouch **300** through fluid tube **302,** which can be fluidly connectable to a fluid line **301.** The fluid line **301** can connect to a peritoneal dialysis fluid generation system for generation of peritoneal dialysis fluid from a concentrate in concentrate pouch **300.** The fluid tube **302** can extend upwardly into an interior space of the concentrate pouch **300** and can include an integrated venturi system **307** within the fluid tube **302** for faster dissolution of solid material and formation of a homogeneous solution inside the concentrate pouch **300.** The integrated venturi system **307** can include a venturi tube (not shown) forming a constriction inside the fluid tube **302.** The constriction inside the venturi tube can vary pressures and fluid flow rates. The concentrate pouch **300** can include an opening **303** in fluid tube **302** downstream of the integrated venturi system **307.** The constriction can increase the speed of the fluid to create a local pressure drop that provides a suction force for drawing material from an interior of the concentrate pouch **300** through the opening **303.** The concentrate pouch **300** can also have a first inclination **305a** and a second inclination **305b** to encourage material or fluid to move in a downwardly direction when the concentrate pouch **300** is upright. The concentrate pouch **300** can also have an interior support structure **308** welded to an interior side of the concentrate pouch **300** to support the fluid tube **302.** The support structure **308** can be formed into a rigid V-shaped support made of suitable materials and can be affixed to interior surfaces of the concentrate pouch **300.** Any other suitable support structure and techniques for rigidly positioning the fluid tube **302** upright inside the interior of the concentrate pouch **300** can be formed by those of ordinary skill.

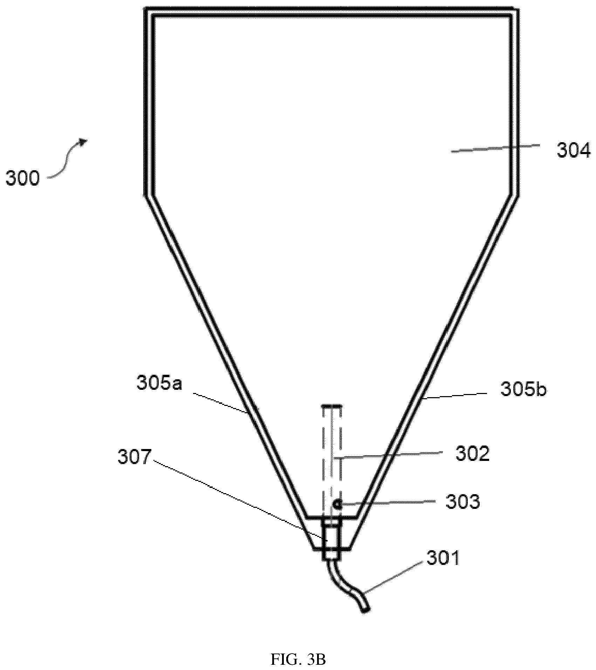

FIG. 3B is an exterior side view of the concentrate pouch **300** showing an exterior side **304** of the peritoneal dialysis concentrate pouch **300.** The interior side **306** of the same exterior side **304** of the concentrate pouch **300** is depicted in FIG. 3A. As shown in FIG. 3B, the first inclination **305a** and the second inclination **305b** taper into a cone-shape to encourage material or fluid to move in a downwardly direction towards the fluid tube **302** at a bottom portion of the concentrate pouch **300.** The first inclination **305a** and the second inclination **305b** can collect powders at the bottom of the concentrate pouch **300** to assist dissolution or fluid flow. Further, the first inclination **305a** and the second inclination **305b** can help to avoid the creation of no-flow zones within the concentrate pouch **300.** In certain embodiments, the first inclination **305a** and the second inclination **305b** can each have an angle of inclination ranging from 20°-60° as measured from the horizontal axis at the bottom portion of the concentrate pouch **300.**

The fluid tube **302** shown in dashed line in FIG. 3B can connect to fluid line **301** of a peritoneal dialysis fluid generation system by any means known in the art. For example, a bottom section of the fluid inlet tube **302** can include a capped and threaded portion that can mate with a threaded portion on a fluid line connected to a peritoneal dialysis generation system. Alternatively, the fluid inlet tube **302** can include a rigid connector for connection to the peritoneal dialysis generation system. A flap (not shown in FIG.'s 3A-B) can cover the rigid connector prior to connection to separate the contents of the concentrate pouch **300** from the outside environment. Alternatively, a peelable layer can be placed on the bottom of fluid inlet tube **302** that can be removed prior to connection.

Experiments demonstrated that sodium bicarbonate in a concentrate pouch did not dissolve in less than 10 minutes at a flow rate of 60 ml/min into the concentrate pouch without a venturi system. Using a venturi system at a water inlet temperature of 48°C, the bicarbonate was completely dissolved in 2.24 minutes providing a final solution temperature of 45°C. Table 2 shows results for a similar experiment using dextrose in a concentrate pouch without a venturi system wherein the time dissolve by mixing took 130 minutes with a temperature after dissolution of 24.5°C. Although dissolution tests are influenced by the final concentration, temperature, and other parameters, the experiments show that the venturi system can speed dissolution under the tested conditions. As shown in Table 2, dextrose took longer to dissolve or mix at lower temperatures. Without a venturi system, at 24.5 °C, the dextrose took 130 minutes to dissolve, at 34.6°C the dextrose took 45 minutes to dissolve and at 40°C the dextrose took 30 minutes to dissolve. However, when using a venturi system, the dextrose was dissolved in 4 minutes at 49°C. The venturi system significantly reduced the time of dissolution for all possible powders tested. Alternatively, any other type of concentrate container can be used, including solid or flexible containers and concentrate bags with or without an integrated venturi system.

**Table 2**

| Time to Dissolve/Mix (min) | Temperature after Dissolution/Mixing (°C) |
|---|---|
| 130 | 24.5 |
| 45 | 34.6 |
| 30 | 40 |

Table 3 provides non-limiting examples of combinations and sizes of concentrate containers that can be used. One of skill in the art will understand that alternative arrangements of materials across one or more concentrate containers can be included, and Table 3 is provided for illustrative purposes only.

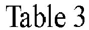

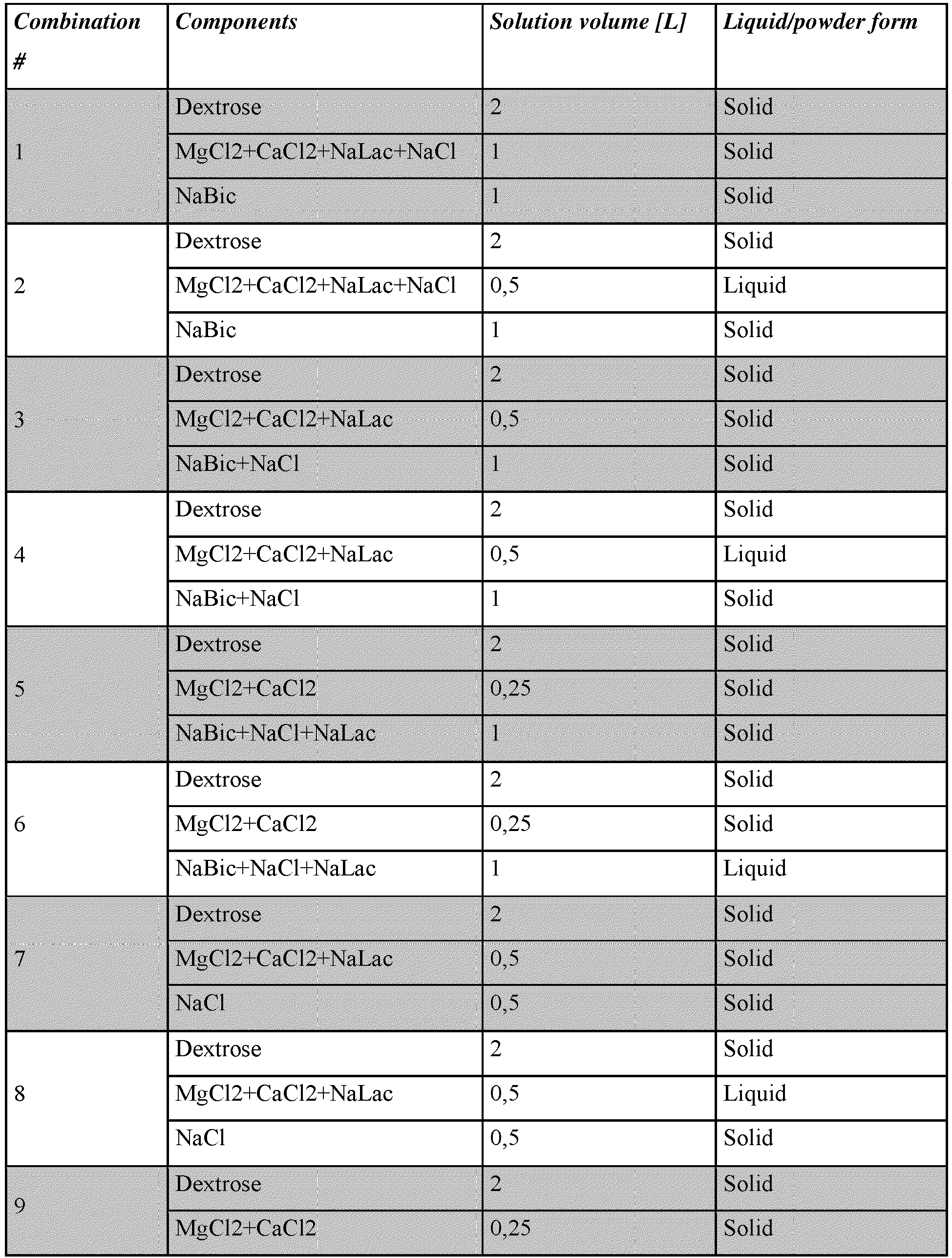

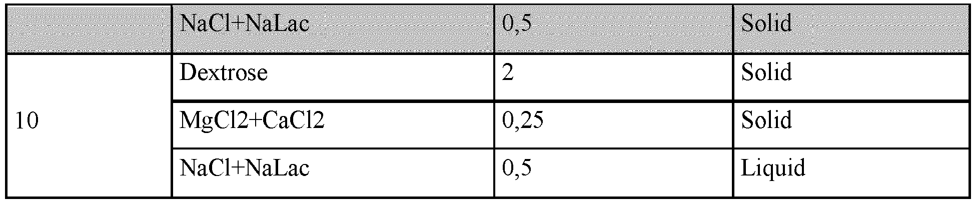

The peritoneal dialysis fluid generated using the concentrate pouches can include sodium chloride, sodium lactate, magnesium chloride, calcium chloride, dextrose, and optionally sodium bicarbonate. However, in certain embodiments, the lactate can be eliminated and only sodium bicarbonate, or the sodium bicarbonate eliminated and only sodium lactate used. As shown in Table 1, to avoid precipitate formation, wither water or dextrose must be pumped to the mixing container between the sodium bicarbonate concentrate and the magnesium and calcium concentrate. To avoid precipitation, the magnesium and calcium can be separated from the sodium bicarbonate, as shown in combinations 1-6 in Table 3. In each of the combinations listed in Table 3, dextrose is provided in a concentrate container without any other components. Providing dextrose without additional components can allow for the dextrose concentration of the resulting peritoneal dialysis fluid to be set independently of the sodium, magnesium, calcium, or buffer concentrations, allowing therapy to be customized based on the medical prescription. However, in certain embodiments, the dextrose can be included in a single concentrate container with one or more additional components. While bicarbonate can be separated from the magnesium and calcium, any 2 or more concentrate containers can be used with the other components in any arrangement. The actual combination of components in each concentrate container used can depend on the needs of the user, and whether the dialysis fluid concentration of any components needs to be set independently of any other components.

One skilled in the art will understand that various combinations and/or modifications and variations can be made in the described systems and methods depending upon the specific needs for operation. Various aspects disclosed herein may be combined in different combinations than the combinations specifically presented in the description and accompanying drawings. Moreover, features illustrated or described as being part of an aspect of the disclosure may be used in the aspect of the disclosure, either alone or in combination, or follow a preferred arrangement of one or more of the described elements. Depending on the example, certain acts or events of any of the processes or methods described herein may be performed in a different sequence, may be added, merged, or left out altogether (e.g., certain described acts or events may not be necessary to carry out the techniques). In addition, while certain aspects of this disclosure are described as performed by a single module or unit for purposes of clarity, the techniques of this disclosure may be performed by a combination of units or modules associated with, for example, a medical device.

## Claims

1. A system, comprising:
a peritoneal dialysis fluid generation flow path, the peritoneal dialysis fluid generation flow path fluidly connectable to a water source;
one or more concentrate containers fluidly connectable to the peritoneal dialysis fluid generation flow path, the one or more concentrate containers comprising: at least a concentrate container containing at least one of calcium or magnesium, at least a concentrate container containing at least dextrose, and at least a concentrate container containing at least sodium bicarbonate; wherein sodium chloride is present in at least one concentrate container; and
a control system programmed to pump water from the water source into each of the one or more concentrate containers to generate one or more concentrates, the control system also being programmed to subsequently pump the one or more generated concentrates into the mixing container, wherein the control system is programmed to sequentially pump a dextrose concentrate from the concentrate container containing at least dextrose into the mixing container in between:
pumping an ion concentrate from the concentrate container containing at least calcium or magnesium into the mixing container and pumping a sodium bicarbonate concentrate from the concentrate container containing sodium bicarbonate into the mixing container, or in between:
pumping a sodium bicarbonate concentrate from the concentrate container containing sodium bicarbonate into the mixing container and pumping an ion concentrate from the concentrate container containing calcium or magnesium into the mixing container.

2. The system of claim 1, further comprising one or more filters in the peritoneal dialysis fluid generation flow path.

3. The system of claim 1, wherein the concentrate container containing sodium bicarbonate further comprises sodium lactate.

4. The system of claims 1 or 3, wherein the concentrate container containing sodium bicarbonate further comprises sodium chloride.

5. The system of any of claims 1-4, wherein at least one of the concentrate containers initially contains a solid substance.

6. The system of any of claims 1 to 4, wherein at least one of the concentrate containers initially contains a liquid or aqueous solution.

7. The system of any of claims 1-6, further comprising one or more valves directing fluid between the one or more concentrate containers and the mixing container.

8. The system of any of claims 1-6, wherein the water pumped into the one or more concentrate containers is purified water.

9. The system of claim 8, further comprising a water purification module downstream of the water source and in fluid communication with the water source.

10. The system of any of claims 1-6, wherein the water pumped into the one or more concentrate container is sterile water.

11. The system of claim 10 further comprising a water sterilization module downstream of the water source and in fluid communication with the water source.

12. The system of claim 2, wherein the one or more filters is positioned upstream of the mixing container.

13. The system of any of claims 1-6, further comprising a peritoneal dialysis cycler in fluid connection with the mixing container.

14. The system of any of claims 1-6, wherein the one or more concentrate containers is a pouch.

15. A method, comprising the steps of:
pumping water from a water source into one or more concentrate containers to generate a concentrate; the one or more concentrate containers comprising: at least a concentrate container containing at least one of calcium or magnesium, at least a concentrate container containing at least dextrose, and at least a concentrate container containing at least sodium bicarbonate; wherein sodium chloride is present in at least one concentrate container; and
pumping a dextrose concentrate from the concentrate container containing dextrose into the mixing container in between pumping an ion concentrate from the concentrate container containing calcium or magnesium into the mixing container and pumping a sodium bicarbonate concentrate from the concentrate container containing sodium bicarbonate into the mixing container, or
pumping the dextrose concentrate from the concentrate container containing dextrose in between pumping a sodium bicarbonate concentrate from the concentrate container containing sodium bicarbonate into the mixing container and pumping an ion concentrate from the concentrate container containing calcium or magnesium into the mixing container;
optionally further comprising the step of pumping water into the mixing container;
optionally wherein the concentrate container containing sodium bicarbonate further comprises sodium lactate;
optionally wherein the water pumped into the one or more concentrate containers is purified water or wherein the water pumped into the one or more concentrate containers is sterile water;
optionally further comprising the step of pumping water to the mixing container after the step of pumping one or more concentrates from the one or more concentrate containers;
optionally further comprising the step of filtering fluid prior to adding the concentrates to the mixing container;
optionally further comprising the step of pumping fluid inside the mixing container to a peritoneal dialysis cycler; and
optionally wherein the one or more concentrate containers is a pouch.
